(19) Europäisches Patentamt
European Patent Office
Office européen des brevets

(11) EP 4 458 802 A1

(12) EUROPEAN PATENT APPLICATION
published in accordance with Art. 153(4) EPC

(43) Date of publication:
**06.11.2024 Bulletin 2024/45**

(21) Application number: **22826630.0**

(22) Date of filing: **08.10.2022**

(51) International Patent Classification (IPC):
***C07C 68/08*** (2006.01) ***C07C 68/065*** (2020.01)
***C07C 69/96*** (2006.01)

(52) Cooperative Patent Classification (CPC):
Y02P 20/10

(86) International application number:
**PCT/CN2022/123773**

(87) International publication number:
**WO 2023/124348 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.12.2021 CN 202111670151**

(71) Applicant: **Shenzhen Capchem Technology Co., Ltd.**
**Shenzhen, Guangdong 518118 (CN)**

(72) Inventors:
- **KANG, Yuanyuan**
  **Shenzhen, Guangdong 518118 (CN)**
- **TU, Pengcheng**
  **Shenzhen, Guangdong 518118 (CN)**
- **ZOU, Xianshuai**
  **Shenzhen, Guangdong 518118 (CN)**
- **TANG, Xiwu**
  **Shenzhen, Guangdong 518118 (CN)**
- **CHEN, Senwei**
  **Shenzhen, Guangdong 518118 (CN)**

(74) Representative: **Isern Patentes y Marcas S.L.**
**Avda. Diagonal, 463 Bis, 2°**
**08036 Barcelona (ES)**

# (54) LINEAR CARBONATE AND PREPARATION METHOD THEREFOR

(57) The present invention relates to the technical field of chemical engineering, and in particular to a linear carbonate and a preparation method thereof, which includes one or more of compounds of structural formula 1 below:

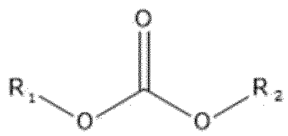

wherein Ri and $R_2$ are respectively selected from one of alkyl groups containing 1-4 carbon atoms;
a hydroxyl concentration of the linear carbonate is no more than 100 ppm, and a free acid conversion rate of a solution with a concentration of 1 mol/L as formulated from the linear carbonate and lithium hexafluorophosphate is less than 1.2 after storage under 25°C for 30 days. An acidity conversion rate was reduced when lithium hexafluorophosphate is dissolved in the linear carbonate by controlling the hydroxyl concentration, the energy density, discharge capacity, safety performance and service life of a battery can be improved when it's electrolyte solution contains the linear carbonate.

EP 4 458 802 A1

## Description

### TECHNICAL FIELD

The present invention relates to the technical field of chemical engineering, and in particular to a linear carbonate and a preparation method thereof.

### BACKGROUND

A linear carbonate is an excellent solvent for an electrolyte solution of a lithium-ion battery because of its low viscosity, high dielectric constant and strong solubility for a lithium salt. It can improve the energy density and discharge capacity of the battery, and further improve the safety performance and service life of the battery. Therefore, it is urgent to develop a linear carbonate with a more stable performance.

### SUMMARY

In order to solve the aforementioned technical problem, the present invention provides a linear carbonate and a preparation method thereof. This method reduces an acidity conversion rate after lithium hexafluorophosphate is dissolved in the linear carbonate by controlling the hydroxyl concentration in the linear carbonate, so that a formulated electrolyte solution of a lithium battery can avoid the influence on the performance of the battery due to the increase in acidity.

The present invention provides a linear carbonate, which includes one or more of compounds of structural formula 1 below:

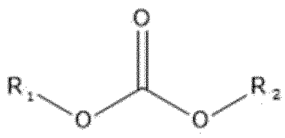

structural formula 1

wherein R1 and R2 are respectively selected from one of alkyl groups containing 1-4 carbon atoms; a hydroxyl concentration of the linear carbonate is no more than 100 ppm, and a free acid conversion rate of a solution with a concentration of 1 mol/L as formulated from the linear carbonate and lithium hexafluorophosphate is less than 1.2 after sealed storage under a condition of a constant temperature of 25°C for 30 days.

In some embodiments of the present invention, the compound of structural formula 1 is selected from one or more of the following compounds:

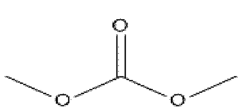 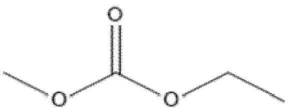 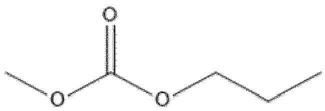

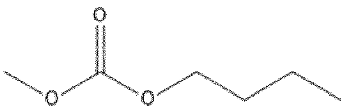 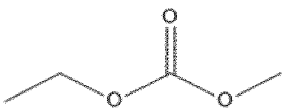 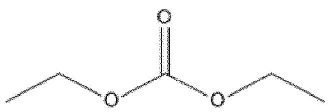

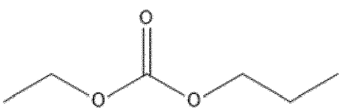 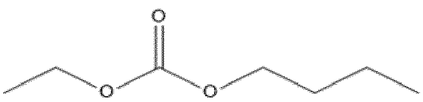 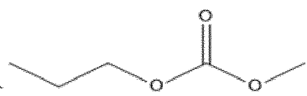

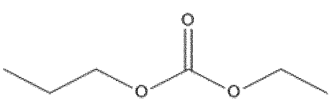 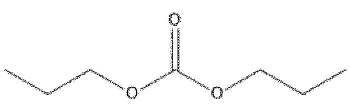 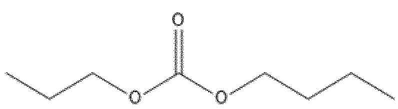

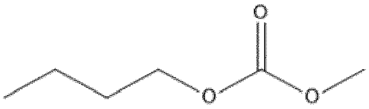 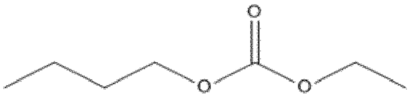

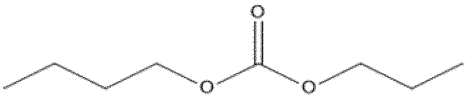 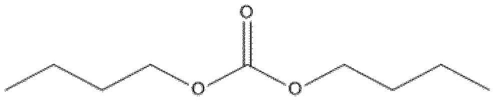

In some embodiments of the present invention, the hydroxyl concentration of the linear carbonate is no more than 60 ppm.

In a second aspect, the present invention provides a method for preparing the aforementioned linear carbonate, which includes the following preparation steps:

step 1: mixing ethylene carbonate with a catalyst and then adding the mixture into a reaction rectification tower, and introducing an alcohol compound for transesterification;
step 2: introducing a condensed fraction collected at a top of the reaction rectification tower into a refining tower, controlling a temperature at a top of the refining tower to be 20~40°C higher than that at a bottom of the reaction rectification tower, and side withdrawing the linear carbonate;
step 3: introducing the linear carbonate obtained in the step 2 into a melting crystallizer after passing through an adsorbent for adsorption, cooling to -50~70°C at a cooling rate of 1-3°C/h, and then keeping at this temperature for 1-5 h; and
step 4: purifying a crystal obtained in the step 3 by sweating at a controlled sweating temperature of 1-5°C and a sweating ratio of 3-7% of a mass of the crystal, separating sweating liquor, and heating the remaining crystal to melting to obtain the linear carbonate.

In some embodiments of the present invention, in the step 1, the feeding molar ratio of the ethylene carbonate to the alcohol compound is 1:2~1:10; and
preferably, the feeding molar ratio of the ethylene carbonate to the alcohol compound is 1:2~1:6.

The alcohol compound is selected from at least one of methanol, ethanol, propanol and butanol.

Preferably, the alcohol compound is selected from two of methanol, ethanol, propanol and butanol.

In some embodiments of the present invention, the transesterification is conducted at a reaction pressure of 0.1~0.25 MPa and a reaction temperature of 50~120°C.

Preferably, the transesterification is conducted at a reaction pressure of 0.21 MPa and a reaction temperature of 110°C.

In some embodiments of the invention, the catalyst is selected from one or more of sodium methoxide, potassium carbonate, sodium carbonate, imidazole ionic liquid, quaternary ammonium ionic liquid and quaternary phosphonium ionic liquid.

In some embodiments of the invention, the adsorbent is selected from one or more of a molecular sieve, activated carbon and a cation exchange resin.

Compared with the prior art, the present invention has the following advantages:

The present invention provides a linear carbonate, wherein the hydroxyl concentration in the linear carbonate is no more than 100 ppm, and a free acid conversion rate of a solution with a concentration of 1 mol/L as formulated from the linear carbonate and lithium hexafluorophosphate is less than 1.2 after sealed storage under a condition of a constant temperature of 25°C for 30 days. The present invention reduces an acidity conversion rate after lithium hexafluorophosphate is dissolved in the linear carbonate by controlling the hydroxyl concentration in the linear carbonate, so that when the linear carbonate is used as a solvent of an electrolyte solution, the energy density and discharge capacity of a battery can be improved, and further the safety performance and service life of the battery can be improved.

## DETAILED DESCRIPTION OF EMBODIMENTS

The following clearly and completely describes the technical solutions in the embodiments of the present invention with reference to the embodiments of the present invention. Apparently, the described embodiments are merely a part rather than all of the embodiments of the present invention. All other embodiments obtained by those of ordinary skills in the art based on the embodiments of the present invention without creative efforts shall fall within the claimed scope of the present invention.

The present invention will be described in detail through examples hereafter. The structural formulas of the compounds shown in Table 1 are all products that can be prepared by the preparation methods described in the examples

of the present invention.

Table 1

| | | |
|---|---|---|
| Compound 1 | Compound 2 | Compound 3 |
| 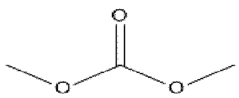 | 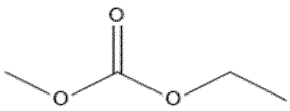 | 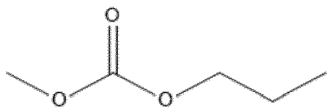 |
| Compound 4 | Compound 5 | Compound 6 |
| 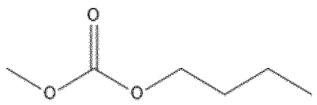 | 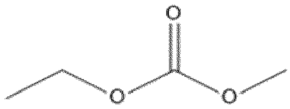 | 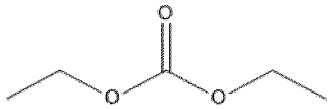 |
| Compound 7 | Compound 8 | Compound 9 |
| 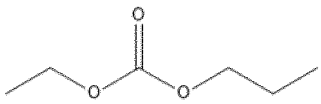 | 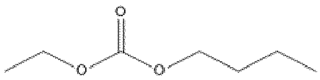 | 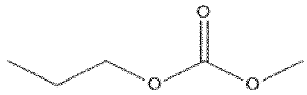 |
| Compound 10 | Compound 11 | Compound 12 |
| 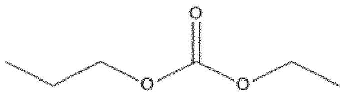 | 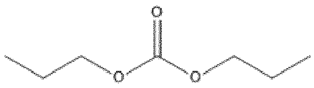 | 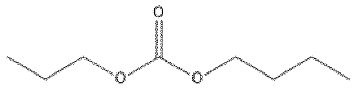 |
| Compound 13 | Compound 14 | Compound 15 |
| 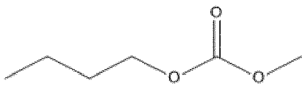 | 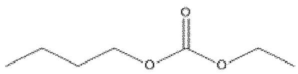 | 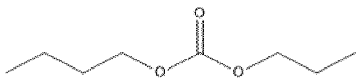 |
| Compound 16 | | |
| 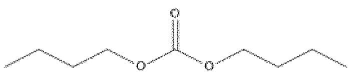 | | |

Example 1

The preparation of a linear carbonate, including the following steps:

step 1: ethylene carbonate was mixed with potassium carbonate, and then introduced into a reaction rectification tower, and then methanol was introduced for transesterification at a reaction pressure of 0.15 MPa and a reaction temperature of 70°C, with the molar ratio of ethylene carbonate to methanol being 1:2;
step 2: a condensed fraction collected at a top of the reaction rectification tower was introduced into a refining tower, a temperature at a top of the refining tower was controlled to be 20°C higher than that at a bottom of the reaction rectification tower, a light component was withdrawn from the top of the refining tower, a heavy component was withdrawn from the bottom of the refining tower, and dimethyl carbonate was withdrawn from the side line of the refining tower;
step 3: the dimethyl carbonate obtained in the step 2 was introduced into a melting crystallizer after adsorption by activated carbon, cooled to -50°C at a cooling rate of 3°C/h, and kept at this temperature for 1 h; and
step 4: the dimethyl carbonate crystal obtained in the step 3 was heated to 1°C for purification by sweating at a controlled sweating ratio of 3%, the sweating liquor was separated, and the remaining crystal was heated to melting to obtain electronic-grade dimethyl carbonate.

The tested hydroxyl solubility of the electronic-grade dimethyl carbonate was 80 ppm, and the tested acidity conversion rate of a formulated solution of lithium hexafluorophosphate in it was 1.15.

Example 2

The preparation of a linear carbonate, including the following steps:

step 1: ethylene carbonate was mixed with potassium carbonate, and then introduced into a reaction rectification tower, and then ethanol was introduced for transesterification at a reaction pressure of 0.2 MPa and a reaction temperature of 95°C, with the molar ratio of ethylene carbonate to ethanol being 1:3;
step 2: a condensed fraction collected at a top of the reaction rectification tower was introduced into a refining tower, a temperature at a top of the refining tower was controlled to be 40°C higher than that at a bottom of the reaction rectification tower, a light component was withdrawn from the top of the refining tower, a heavy component was withdrawn from the bottom of the refining tower, and diethyl carbonate was withdrawn from the side line of the refining tower;
step 3: the diethyl carbonate obtained in the step 2 was introduced into a melting crystallizer after adsorption by activated carbon, cooled to 20°C at a cooling rate of 1.5°C/h, and kept at this temperature for 3 h; and
step 4: the diethyl carbonate crystal obtained in the step 3 was heated to 4°C for purification by sweating at a controlled sweating ratio of 5%, the sweating liquor was separated, and the remaining crystal was heated to melting to obtain electronic-grade diethyl carbonate. The testing results of the product were filled into Table 2.

Example 3

The preparation of a linear carbonate, including the following steps:

step 1: ethylene carbonate was mixed with sodium methoxide, and then introduced into a reaction rectification tower, and then methanol was introduced from an upper end of the rectification tower and ethanol was introduced from the lower end of the rectification tower for transesterification at a reaction pressure of 0.21 MPa and a reaction temperature of 110°C, with the molar ratio of ethylene carbonate to methanol and ethanol being 1:4:2;
step 2: a condensed fraction collected at a top of the reaction rectification tower was introduced into a refining tower, a temperature at a top of the refining tower was controlled to be 30°C higher than that at a bottom of the reaction rectification tower, a light component was withdrawn from the top of the refining tower, a heavy component was withdrawn from the bottom of the refining tower, and methyl ethyl carbonate was withdrawn from the side line of the refining tower;
step 3: the methyl ethyl carbonate obtained in the step 2 was introduced into a melting crystallizer after adsorption by activated carbon, cooled to -10°C at a cooling rate of 2°C/h, and kept at this temperature for 2 h; and
step 4: the methyl ethyl carbonate crystal obtained in the step 3 was heated to 2°C for purification by sweating at a controlled sweating ratio of 4%, the sweating liquor was separated, and the remaining crystal was heated to melting to obtain electronic-grade methyl ethyl carbonate. The testing results of the product were filled into Table 2.

Comparative Example 1

The preparation of a linear carbonate, including the following steps:

step 1: ethylene carbonate was mixed with potassium carbonate, and then introduced into a reaction rectification tower, and then methanol was introduced for transesterification at a reaction pressure of 0.15 MPa and a reaction temperature of 70°C, with the molar ratio of ethylene carbonate to methanol being 1:2;
step 2: a condensed fraction collected at a top of the reaction rectification tower was introduced into a refining tower, a temperature at a top of the refining tower was controlled to be 45°C higher than that at a bottom of the reaction rectification tower, a light component was withdrawn from the top of the refining tower, a heavy component was withdrawn from the bottom of the refining tower, and dimethyl carbonate was withdrawn from the side line of the refining tower;
step 3: the dimethyl carbonate obtained in the step 2 was introduced into a melting crystallizer after adsorption by activated carbon, cooled to -55°C at a cooling rate of 4°C/h, and kept at this temperature for 1 h; and
step 4: the dimethyl carbonate crystal obtained in the step 3 was heated to 1°C for purification by sweating at a controlled sweating ratio of 2%, the sweating liquor was separated, and the remaining crystal was heated to melting to obtain electronic-grade dimethyl carbonate. The testing results of the product were filled into Table 2.

Comparative Example 2

The preparation of a linear carbonate, including the following steps:

step 1: ethylene carbonate was mixed with sodium methoxide, and then introduced into a reaction rectification tower, and then methanol was introduced from an upper end of the rectification tower and ethanol was introduced from the lower end of the rectification tower for transesterification at a reaction pressure of 0.21 MPa and a reaction temperature of 110°C, with the molar ratio of ethylene carbonate to methanol and ethanol being 1:4:2;
step 2: a condensed fraction collected at a top of the reaction rectification tower was introduced into a refining tower, a temperature at a top of the refining tower was controlled to be 15°C higher than that at a bottom of the reaction rectification tower, a light component was withdrawn from the top of the refining tower, a heavy component was withdrawn from the bottom of the refining tower, and methyl ethyl carbonate was withdrawn from the side line of the refining tower;
step 3: the methyl ethyl carbonate obtained in the step 2 was introduced into a melting crystallizer after adsorption by activated carbon, cooled to -10°C at a cooling rate of 2°C/h, and kept at this temperature for 2 h; and
step 4: the methyl ethyl carbonate crystal obtained in the step 3 was heated to 6°C for purification by sweating at a controlled sweating ratio of 8%, the sweating liquor was separated, and the remaining crystal was heated to melting to obtain electronic-grade methyl ethyl carbonate. The testing results of the product were filled into Table 2.

Table 2

| Group | Product | Hydroxyl concentration | Acidity conversion rate |
|---|---|---|---|
| Example 1 | Dimethyl carbonate | 80 ppm | 1.15 |
| Example 2 | Diethyl carbonate | 50 ppm | 1.13 |
| Example 3 | Methyl ethyl carbonate | 30 ppm | 1.08 |
| Comparative Example 1 | Dimethyl carbonate | 130 ppm | 1.5 |
| Comparative Example 2 | Methyl ethyl carbonate | 120 ppm | 1.47 |

The specific testing methods of the hydroxyl concentration in the linear carbonate obtained by the present invention and the acidity conversion rate of the linear carbonate were as follows:

1. Method of testing total hydroxyl concentration:

1.1 A hydroxyl-containing substance was identified in a compound 1 by a gas chromatograph-mass spectrometer, then the hydroxyl-containing substance in the compound 1 was quantified by gas chromatography and the moisture content in the compound 1 was tested by a moisture tester, and subsequently the total hydroxyl concentration was converted according to the concentration and moisture content of each hydroxyl substance.
1.2 The moisture content W ($H_2O$) in the compound 1 was tested in ppm according to the method in national standard GB/T 6284-2006;
1.3 If it was identified that the hydroxyl-containing substance in the compound was only a lower alcohol (with a carbon number $\leq 4$, an unknown concentration, recorded as $W_0$ in ppm), a spiked test can be carried out in the following manner.
$m_0 \times g$ of the compound 1 was taken as the solvent, and then added with miyg of the lower alcohol standard substance (of analytically pure and above), so that $m_1/m_0 = 0.05\%$, and a spiked sample of the linear carbonate was formulated at a concentration of the lower alcohol $\approx W_0 + 500$ ppm.
Then, it was allowed that $m_1/m_0 = 0.02\%$, 0.01%, 0.005% and 0.002% sequentially for formulation sequentially, so as to obtain spiked samples of the linear carbonate with concentrations of the lower alcohol of ($W_0$ + 200 ppm), ($W_0$ + 100 ppm), ($W_0$ + 50 ppm), and ($W_0$ + 20 ppm) respectively.
1.4 The gas chromatograph was set according to the following parameters, and then the aforementioned spiked samples of the carbonates were tested.

Model of chromatographic column: SE-54 capillary column

An injection port temperature was 200°C, an injection volume was 1 uL, a split ratio was 200: 1, and a detector temperature was 250°C
Programmed temperature increasing: the starting temperature was 100°C and kept for 4 min, and then increased at 25°C/min to 250°C, and this temperature was kept for 10 min.
1.5 The peak area A of the lower alcohol was taken as a vertical coordinate and the additional spiked concentration (20, 50, 100, 200, 500 ppm) of each lower alcohol was taken as a horizontal coordinate, and linear fitting was conducted to obtain a curve with an equation of $y = ax + b$ (wherein a and b > 0), and thus the concentration of the lower alcohol in the compound 1 was $W_0 = b/a$, in ppm.

1.6 If the compound 1 contained a variety of hydroxyl-containing substances such as A, B, C... etc., and the concentrations $WA_0$, $WB_0$, $WC_0$ ...of A, B, C...etc. could be determined by the aforementioned methods, then the total hydroxyl concentration in the compound 1 was:

$$W(OH.ppm) = 2W(H_2O) + \sum_A^{A,B,C\dots} \frac{17*x_A*W_{A0}}{M_A} = 2W(H_2O) + \frac{17*x_A*W_{A0}}{M_A} + \frac{17*x_B*W_{B0}}{M_B} + \frac{17*x_C*W_{C0}}{M_C} + \cdots$$

Wherein $M_A$, $M_B$, $M_C$... were the relative molecular masses of A, B, C... etc., $X_A$, $X_B$, $X_C$...were the number of hydroxyl groups in the molecules A, B, C...etc. and $W(H_2O)$ was the moisture content in the linear carbonate.

2. Method of testing acidity conversion rate f after dissolution of lithium salt:

2.1 Dissolution conditions: the refined compound 1 (with the quality recorded as $m_2$) was taken, sealed and frozen below -10°C for 1 h, then added with about 1/9 of its own mass of electronic-grade lithium hexafluorophosphate (with a mass recorded as $m_3$, and a free acid content less than 50 ppm) in a glove box (with a moisture content of less than 10 ppm, and an oxygen content of less than 20 ppm), and shaken well to obtain a solution of 10% lithium hexafluorophosphate dissolved in the linear carbonate, and the accurate concentration percentage of lithium hexafluorophosphate was recorded $x=\frac{m_3}{m_2+m_3} \times 100\%$.

2.2 Storage conditions: the formulated solution of the compound 1 in which 10% lithium hexafluorophosphate was dissolved, was transferred into a clean and dry aluminum flask and sealed. Then it was stored in a thermostat at 25°C for 30 d. After 30 d, it was sampled in a glove box and determined for free acid.

2.3 Method of testing free acid of lithium hexafluorophosphate and lithium hexafluorophosphate solution:

2.3.1 Reagents: anhydrous acetonitrile (of analytically pure and above, and with a moisture content ≤ 20 ppm), a methyl red indicator (0.1%Wt anhydrous acetonitrile), 0.01 mol/L of triethylamine-anhydrous acetonitrile titrant, triethylamine (of analytically pure and above), potassium hydrogen phthalate (standard reagent);

2.3.2 Formulation of triethylamine-anhydrous acetonitrile titrant and standardization method

2.3.2.1 1.01 g of triethylamine was weighed, diluted with anhydrous acetonitrile, transferred into a 1,000 mL volumetric flask, and brought to a constant volume with anhydrous acetonitrile to obtain a 0.01 mol/L triethylamine-anhydrous acetonitrile solution (formulated and used in a glove box);

2.3.2.2 A small amount of potassium hydrogen phthalate was taken as the standard reagent, and oven dried at 110°C for 2 h for later use;

2.3.2.3 0.0204 g of potassium hydrogen phthalate (with its accurate mass recorded as $m_4$, in g, accurating to the 4th decimal place), dissolved by addition of ultrapure water, added dropwise with 3-5 drops of a methyl red-anhydrous acetonitrile solution as the indicator, and titrated with a certain amount of the 0.01 mol/L triethylamine-anhydrous acetonitrile solution taken out of the glove box until the color of the solution changed from red to yellow, and the volume of the consumed triethylamine solution (recorded as $V_1$, in mL) was recorded. Parallel experiments were made with the requirement that the difference between two results was no more than 10% of the average of the two results;

2.3.2.4 Then the concentration of the triethylamine-anhydrous acetonitrile solution was calculated according to the following equation:

$$c = \frac{m_4 \times 4.897}{V_1}, \text{ in mol/L}$$

in mol/L.

2.3.3 Method of titrating free acid content in lithium hexafluorophosphate and lithium hexafluorophosphate solution

2.3.3.1 In a glove box, about 20 g of anhydrous acetonitrile was taken, added dropwise with 3-5 drops of the indicator of 0.1% methyl red-anhydrous acetonitrile, added with the standardized triethylamine-anhydrous acetonitrile titrant until the indicator turned yellow (the addition amount was not recorded), then added with 10-20 g of lithium hexafluorophosphate or a lithium hexafluorophosphate solution that had been stored for 30 d (with its accurate mass recorded as $m_5$, in g, accurating to the 2nd decimal place), titrated with the triethylamine-anhydrous acetonitrile solution until the solution just turned yellow again, and the volume of the consumed triethylamine-anhydrous acetonitrile titrant ($V_2$, in mL, accurating to the 2nd decimal place) was recorded. Parallel tests were made with the requirement that the difference

between two determination results was no more than 20% of the average of the two results;

2.3.3.2 Then the free acid content in lithium hexafluorophosphate or the lithium hexafluorophosphate solution after storage was (in ppm, by HF):

$$W(HF)_0=\frac{20000cV_2}{m_5};$$

The content of free acid in the lithium hexafluorophosphate solution after storage was (in ppm, by HF):

$$W(HF)_1=\frac{20000cV_2'}{m_5'};$$

2.3.4 Calculation formula of acidity conversion rate:

$$f=\frac{W(HF)_1-x*W(HF)_0}{W(OH)\times(1-x)}\times 100\%$$

For the linear carbonate of the present invention, by controlling the hydroxyl concentration in the linear carbonate, it can be ensured that after stored for a long period, the linear carbonate can have a small acidity conversion rate and more stable product properties after dissolution of a lithium salt in it.

The present invention has been further described by means of specific examples hereinabove, but it should be understood that the specific description here should not be construed as limiting the essence and scope of the present invention, and various modifications made by those of ordinary skills in the art to the aforementioned examples after reading the present specification are all within the claimed scope of the present invention.

## Claims

1. A linear carbonate, comprising one or more of compounds of structural formula 1 below:

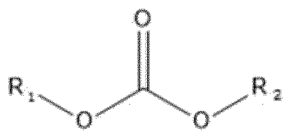

structural formula 1

wherein $R_1$ and $R_2$ are respectively selected from one of alkyl groups containing 1-4 carbon atoms; a hydroxyl concentration of the linear carbonate is no more than 100 ppm, and a free acid conversion rate of a solution with a concentration of 1 mol/L as formulated from the linear carbonate and lithium hexafluorophosphate is less than 1.2 after sealed storage under a condition of a constant temperature of 25°C for 30 days.

2. The linear carbonate according to claim 1, wherein the compound of structural formula 1 is selected from one or more of the following compounds:

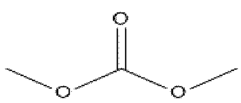 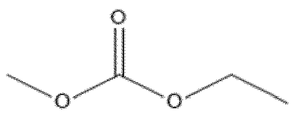 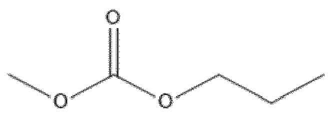

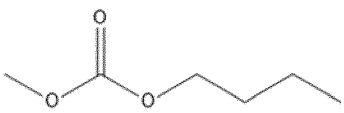 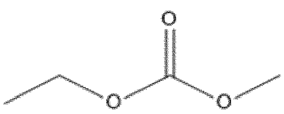 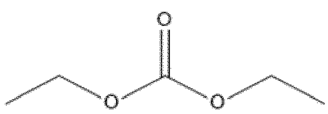

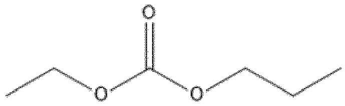 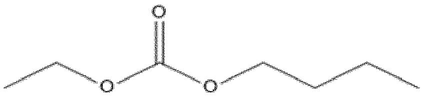 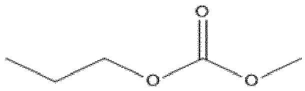

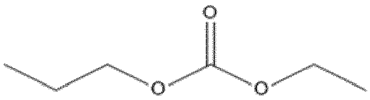 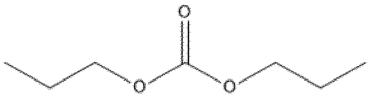 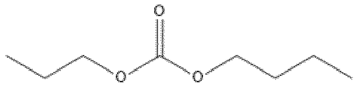

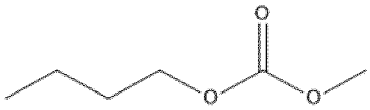 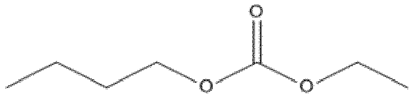

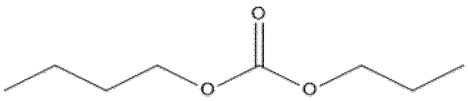 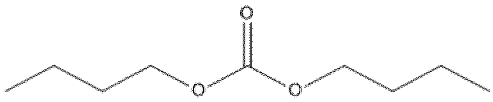

**3.** The linear carbonate according to claim 1 or 2, wherein the hydroxyl concentration of the linear carbonate is no more than 60 ppm.

**4.** A method for preparing the linear carbonate according to any one of claims 1-3, comprising the following preparation steps:

step 1: mixing ethylene carbonate with a catalyst and then adding the mixture into a reaction rectification tower, and introducing an alcohol compound for transesterification;
step 2: introducing a condensed fraction collected at a top of the reaction rectification tower into a refining tower, controlling a temperature at a top of the refining tower to be 20~40°C higher than that at a bottom of the reaction rectification tower, and side withdrawing the linear carbonate;
step 3: introducing the linear carbonate obtained in the step 2 into a melting crystallizer after passing through an adsorbent for adsorption, cooling to (-50)-70°C at a cooling rate of 1-3°C/h, and then keeping at this temperature for 1-5 h; and
step 4: purifying a crystal obtained in the step 3 by sweating at a controlled sweating temperature of 1-5°C and a sweating ratio of 3-7% of a mass of the crystal, separating sweating liquor, and heating the remaining crystal to melting to obtain the linear carbonate.

**5.** The method for preparing the linear carbonate according to claim 4, wherein in the step 1, the feeding molar ratio of the ethylene carbonate to the alcohol compound is 1:2~1:10; and
preferably, the alcohol compound is selected from at least one of methanol, ethanol, propanol and butanol.

**6.** The method for preparing the linear carbonate according to claim 4, wherein in the step 1, the transesterification is conducted at a reaction pressure of 0.1~0.25 MPa and a reaction temperature of 50~120°C.

**7.** The method for preparing the linear carbonate according to claim 4, wherein the catalyst is selected from one or more of sodium methoxide, potassium carbonate, sodium carbonate, imidazole ionic liquid, quaternary ammonium ionic liquid and quaternary phosphonium ionic liquid.

**8.** The method for preparing the linear carbonate according to claim 4, wherein the adsorbent is selected from one or more of a molecular sieve, activated carbon and a cation exchange resin.

**INTERNATIONAL SEARCH REPORT**

International application No.
**PCT/CN2022/123773**

**A. CLASSIFICATION OF SUBJECT MATTER**

C07C 68/08(2006.01)i；C07C 68/065(2020.01)i；C07C 69/96(2006.01)i

According to International Patent Classification (IPC) or to both national classification and IPC

**B. FIELDS SEARCHED**

Minimum documentation searched (classification system followed by classification symbols)

C07C68; C07C69

Documentation searched other than minimum documentation to the extent that such documents are included in the fields searched

Electronic data base consulted during the international search (name of data base and, where practicable, search terms used)

CNTXT; ENTXT; ENTXTC; DWPI; STN; CNKI: 深圳新宙邦科技股份有限公司, 酯交换, 碳酸4w 酯, 反应精馏, 侧线, 结晶, 析晶, 发汗, transesterification, carbonate, rectification, distillation, side line, crystallization, crystalling, sweating

**C. DOCUMENTS CONSIDERED TO BE RELEVANT**

| Category* | Citation of document, with indication, where appropriate, of the relevant passages | Relevant to claim No. |
|---|---|---|
| X | CN 109439447 A (SHENYANG UNIVERSITY OF TECHNOLOGY) 08 March 2019 (2019-03-08)<br>claim 3 | 1-3 |
| X | CN 108496272 A (SOLVAY) 04 September 2018 (2018-09-04)<br>claim 10 | 1-3 |
| X | CN 107501096 A (TIANJIN UNIVERSITY OF SCIENCE AND TECHNOLOGY) 22 December 2017 (2017-12-22)<br>claim 1 | 1-3 |
| A | CN 107501096 A (TIANJIN UNIVERSITY OF SCIENCE AND TECHNOLOGY) 22 December 2017 (2017-12-22)<br>claim 1, and description, paragraphs 8-15 | 4-8 |
| X | CN 111454152 A (DONGYING HI-TECH SPRING CHEMICAL INDUSTRY CO., LTD.) 28 July 2020 (2020-07-28)<br>claim 1 | 1-3 |
| A | CN 111454152 A (DONGYING HI-TECH SPRING CHEMICAL INDUSTRY CO., LTD.) 28 July 2020 (2020-07-28)<br>embodiments 2 and 3 | 4-8 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 November 2022** | **17 November 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/123773**

**C. DOCUMENTS CONSIDERED TO BE RELEVANT**

| Category* | Citation of document, with indication, where appropriate, of the relevant passages | Relevant to claim No. |
|---|---|---|
| X | CN 111704547 A (TIANJIN LEKE ENERGY SAVING TECHNOLOGY CO., LTD. et al.) 25 September 2020 (2020-09-25)<br>claim 1 | 1-3 |
| A | CN 111704547 A (TIANJIN LEKE ENERGY SAVING TECHNOLOGY CO., LTD. et al.) 25 September 2020 (2020-09-25)<br>entire document | 4-8 |
| X | US 4691041 A (TEXACO INC.) 01 September 1987 (1987-09-01)<br>claim 1 | 1-3 |
| A | US 4691041 A (TEXACO INC.) 01 September 1987 (1987-09-01)<br>entire document | 4-8 |
| X | CN 113754540 A (HUIZHOU CAPCHEM CHEMICALS CO., LTD.) 07 December 2021 (2021-12-07)<br>claim 1 | 1-3 |
| A | CN 113754540 A (HUIZHOU CAPCHEM CHEMICALS CO., LTD.) 07 December 2021 (2021-12-07)<br>entire document | 4-8 |
| X | CN 111704545 A (TIANJIN LEKE ENERGY SAVING TECHNOLOGY CO., LTD. et al.) 25 September 2020 (2020-09-25)<br>claim 1 | 1-3 |
| A | CN 111704545 A (TIANJIN LEKE ENERGY SAVING TECHNOLOGY CO., LTD. et al.) 25 September 2020 (2020-09-25)<br>entire document | 4-8 |

Form PCT/ISA/210 (second sheet) (January 2015)

**INTERNATIONAL SEARCH REPORT**
**Information on patent family members**

International application No.
**PCT/CN2022/123773**

| Patent document cited in search report | | | Publication date (day/month/year) | Patent family member(s) | | | Publication date (day/month/year) |
|---|---|---|---|---|---|---|---|
| CN | 109439447 | A | 08 March 2019 | None | | | |
| CN | 108496272 | A | 04 September 2018 | EP | 3369125 | A1 | 05 September 2018 |
| | | | | KR | 20180073640 | A | 02 July 2018 |
| | | | | HU | E051248 | T2 | 01 March 2021 |
| | | | | JP | 2019500716 | A | 10 January 2019 |
| | | | | WO | 2017074556 | A1 | 04 May 2017 |
| | | | | US | 2019058221 | A1 | 21 February 2019 |
| | | | | CA | 3002153 | A1 | 04 May 2017 |
| | | | | PL | 3369125 | T3 | 02 November 2020 |
| CN | 107501096 | A | 22 December 2017 | None | | | |
| CN | 111454152 | A | 28 July 2020 | None | | | |
| CN | 111704547 | A | 25 September 2020 | None | | | |
| US | 4691041 | A | 01 September 1987 | DE | 3781796 | D1 | 22 October 1992 |
| | | | | EP | 0298167 | A1 | 11 January 1989 |
| CN | 113754540 | A | 07 December 2021 | None | | | |
| CN | 111704545 | A | 25 September 2020 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 62842006 T